# EUROPÄISCHE PATENTSCHRIFT

(11) **EP 2 943 242 B1**
(45) Veröffentlichungstag und Bekanntmachung des Hinweises auf die Patenterteilung: **11.01.2017**
(21) Anmeldenummer: 13795495.4
(22) Anmeldetag: 25.11.2013
(51) Int. Cl.: A61M 16/08, A61M 39/08, F16L 11/112, F16L 11/12, F17D 1/02

(54) **FLUIDSCHLAUCHVORRICHTUNG**
FLUID HOSE DEVICE
DISPOSITIF DE TUYAU DE FLUIDE

(30) Priorität: 10.01.2013 EP 13150731
(43) Veröffentlichungstag der Anmeldung: 18.11.2015
(73) Patentinhaber: MEDIN MEDICAL INNOVATIONS GMBH, 82140 Olching (DE)
(72) Erfinder: SCHMITGEN, Paul, 80802 München (DE)
(74) Vertreter: Isarpatent
(86) Internationale Anmeldenummer: PCT/EP2013/074644
(87) Internationale Veröffentlichungsnummer: WO 2014/108239

(56) Entgegenhaltungen:
- EP-A1- 2 514 478
- WO-A1-96/39999
- US-A- 3 858 615
- US-A- 4 745 881
- US-A1- 2006 118 114

## Beschreibung

### GEBIET DER ERFINDUNG

Die vorliegende Erfindung betrifft eine Fluidschlauchvorrichtung, insbesondere für Beatmungsgeräte, gemäß dem Oberbegriff des Anspruchs 1, wie aus der US 3,858,615 A bekannt.

### TECHNISCHER HINTERGRUND

Ein Beatmungsgerät oder Respirator ist eine elektrisch, heute von Mikroprozessoren gesteuerte oder pneumatisch angetriebene Maschine zur Beatmung von Personen mit unzureichender oder ausgesetzter Eigenatmung. Das Atemgas wird meist mit Sauerstoff angereichert. Je nach Anwendungsbereich wird zwischen Notfall-, Intensiv- und Heimrespirator unterschieden. Auch Narkosegeräte sind spezialisierte Beatmungsgeräte.

Bei Beatmungsgeräten werden die Atemgase durch Fluidleitungen zur Person zugeführt und von der Person zurückgeleitet. Solche Fluidleitungen sind idealerweise leicht und biegsam, um für den Patienten den höchstmöglichen Grad an Komfort zu erzielen.

Im Stand der Technik sind dünnwandige Fluidleitungen bekannt, die schraubenförmige Verstärkungsrippen einschließen, die der Leitung eine bessere Widerstandsfähigkeit gegen das Zerdrückt- und Abgeschnürtwerden verleihen sollen, während sie doch eine leichte und biegsame Ausbildung der Fluidleitung ermöglichen.

Die DE 603 02 303 T2 beschreibt eine solche Leitung, umfassend: zumindest ein dünnes Kunststoffband mit einem vorauslaufenden und einem nachlaufenden Seitenrand, wobei das Band schraubenförmig, mit seiner Fläche im Wesentlichen parallel zur Spiralachse angeordnet ist und, außer an seinen Enden, der vorauslaufende Rand jedes Umlaufs des Bands den nachlaufenden Rand eines vorherigen Umlaufs überlappt und der nachlaufende Rand jedes Umlaufs des Bands den vorauslaufenden Rand eines nachfolgenden Umlaufs unterlappt, und einen Verstärkungswulst aus Kunststoff, der in Nachbarschaft des vorauslaufenden Rands und jeweils zwischen sich überlappenden vorauslaufenden und nachlaufenden Rändern angeordnet ist, dadurch gekennzeichnet, dass der überlappende Rand an einem Rand des Wulstes auf das unterlappende Band trifft oder im Wesentlichen darauf trifft.

Die DE 20 2012 007 386 U1 beschreibt ein Wellrohr zur Aufnahme von Versorgungsleitungen einer medizintechnischen Anlage, mit einem wellenförmigen Innenrohr aus einem ersten Material und einem schlauchförmigen Außenmantel aus einem zweiten Material, wobei der Außenmantel zumindest an den Maxima der Wellenberge des Innenrohrs einen Reibungsschluss oder einen Formschluss mit dem Innenrohr bildet.

Die oben beschriebenen Fluidleitungen benötigen viele Verfahrensschritte zu deren Herstellung und sind daher sehr teuer. Ferner weisen diese Fluidleitungen im gewissen Maße eine Knickneigung auf, da diese eine verminderte Formstabilität aufweisen und bei Unterschreitung eines Mindestbiegeradiuses knicken. Zudem sind die oben beschriebenen Fluidleitungen im gewissen Maße inflexibel und starr, was unerwünscht ist.

Dies ist ein Zustand, den es zu verbessern gilt.

### ZUSAMMENFASSUNG DER ERFINDUNG

Vor diesem Hintergrund liegt der vorliegenden Erfindung die Aufgabe zugrunde, eine sehr einfach herstellbare Fluidschlauchvorrichtung zur Verfügung zu stellen, welche eine verminderte Knickneigung aufweist und dennoch flexibel ist. Erfindungsgemäß wird diese Aufgabe durch eine Fluidschlauchvorrichtung mit den Merkmalen des Patentanspruchs 1 gelöst.

Demgemäß ist vorgesehen:
Eine Fluidschlauchvorrichtung, mit einem Fluidschlauch, durch welchen ein Fluid transportierbar ist, mit einer Vielzahl von in Längsrichtung des Fluidschlauches vorgesehenen, voneinander beabstandeten Knickschutz-Erhebungen, welche sich kreisförmig um die Manteloberfläche des Fluidschlauches herum erstrecken, wobei jeweils zwischen zwei benachbarten Knickschutz-Erhebungen eine Gruppe einer Mehrzahl von Verbindungsstegen vorgesehen ist, die die benachbarten Knickschutz-Erhebungen miteinander verbinden, wobei die Gruppen in einem alternierenden Winkelmuster entlang der Längsrichtung des Fluidschlauches angeordnet sind.

Die der vorliegenden Erfindung zugrunde liegende Idee besteht darin, die Formstabilität einer Fluidschlauchvorrichtung durch Knickschutz-Erhebungen, welche sich kreisförmig um die Manteloberfläche eines Fluidschlauches herum erstrecken, zu erhöhen. Eine weitere Steigerung der Formstabiltät wird erfindungsgemäß durch die Verbindungstege erreicht, welche die Knickschutz-Erhebungen miteinander verbinden. Die gewünschte und gesetzlich geforderte Flexibilität und Formstabilität des Fluidschlauches wird zusätzlich dadurch erreicht, dass die einzelnen Gruppen der Verbindungstege in einem alternierenden Muster entlang der Längsrichtung des Fluidschlauches vorgesehen sind. Je nach Anwendungsgebiet und Randbedingungen kann das Muster, in welchen die Verbindungsstege entlang der Längsrichtung des Fluidschlauches vorgesehen sind, angepasst und verändert werden. Zudem ist diese Fluidschlauchvorrichtung besonders einfach herstellbar, da sie eine minimale Anzahl von Elementen benötigt und vorteilhafter Weise mittels eines einfachen Spritzgussverfahrens herstellbar ist.

Vorteilhafte Ausgestaltungen und Weiterbildungen ergeben sich aus den weiteren Unteransprüchen sowie aus der Beschreibung unter Bezugnahme auf die Figuren der Zeichnung.

Gemäß einer Ausführungsform umfassen die Gruppen zwei Verbindungstege, welche um 180° versetzt zueinander angeordnet sind. Gemäß einer weiteren Ausführungsform sind die Gruppen jeweils um 90° Grad winkelversetzt angeordnet. Durch diese Ausbildung ist das Biegeverhalten der Fluidschlauchvorrichtung winkelunabhängig.

Gemäß einer weiteren Ausführungsform umfassen die Gruppen n Verbindungstege, welche um 360°/n versetzt zueinander angeordnet sind, wobei n eine natürliche Zahl größer oder gleich 3 ist. Je nach Randbedingungen und Anwendungsgebiet der Fluidschlauchvorrichtung kann ein Fachmann die Zahl n ändern, um die Flexibilität und Formstabilität der Fluidschlauchvorrichtung zu erhöhen oder zu verringern.

Gemäß einer weiteren vorteilhaften Ausführungsform sind die Gruppen jeweils um 360°/2n winkelversetzt angeordnet, wobei n eine natürliche Zahl größer oder gleich 3 ist.

Gemäß einer weiteren bevorzugten Ausführungsform sind die Knickschutz-Erhebungen und/oder die Verbindungsstege einstückig mit dem Fluidschlauch ausgebildet. Auf diese können die Herstellungskosten für die Fluidschlauchvorrichtung verringert werden, da die Fluidschlauchvorrichtung mittels eines Spritzgussverfahrens herstellbar ist.

Gemäß einer weiteren Ausführungsform der Erfindung sind die Knickschutz-Erhebungen äquidistant und parallel angeordnet sind. Beispielsweise sind die Knickschutz-Erhebungen in einem Abstand von 0,2 mm - 5 mm zueinander angeordnet. Auch der Abstand der Knickschutz-Erhebungen zueinander und die axiale Länge der Verbindungsstege, beispielsweise zwischen 0,1 mm - 3 mm, kann je nach Anwendungsgebiet und gewünschter Formstabilität angepasst werden.

Gemäß einer weiteren Ausführungsform erstrecken sich die Knickschutz-Erhebungen und die Verbindungstege auf eine gleiche Höhe ausgehend von der Manteloberfläche des Fluidschlauches. Beispielsweise erstrecken sich die Knickschutz-Erhebungen und die Verbindungsstege von der Manteloberfläche des Fluidschlauches von 0,2 mm bis zu 2 mm. Auch durch die Höhe der Knickschutz-Erhebungen und der Verbindungstege lassen sich die Formstabilität und das Biegeverhalten der Fluidschlauchvorrichtung beeinflussen.

Gemäß einer weiteren Ausführungsform ist der Fluidschlauch bzw. die Fluidschlauchvorrichtung ein Kunststoffspritzgussteil aus einem thermoplastischen Kunststoff, z. B. TPE-S. Jedoch kann die Fluidschlauchvorrichtung auch aus einem anderen Kunststoff, beispielsweise einem elastomeren Kunststoff oder einem mit Fasern verstärken Kunststoff ausgebildet sein.

Gemäß einer weiteren Ausführungsform ist die Innenseite des Fluidschlauchs im Wesentlichen eben ausgebildet ist. Auf diese Weise kann das Fluid in dem Fluidschlauch besonders gut transportiert werden.

Gemäß einer weiteren Ausführungsform weisen die Verbindungsstege zugfeste Fasern auf. Durch diese Ausbildung kann das Biegeverhalten der Fluidschlauchvorrichtung zusätzlich beeinflusst werden.

Gemäß einer weiteren Ausführungsform ist der Fluidschlauch aus einem ersten Kunststoff ausgebildet ist und die Knickschutz-Erhebungen und die Verbindungsstege aus einem zweiten, vom ersten Kunststoff verschiedenen Kunststoff ausgebildet.

Gemäß einer weiteren Ausführungsform sind die Knickschutz-Erhebungen und die Verbindungsstege derart ausgebildet, dass die Fluidschlauchvorrichtung ein winkelunabhängiges Biegeverhalten aufweist.

Die obigen Ausgestaltungen und Weiterbildungen lassen sich, sofern sinnvoll, beliebig miteinander kombinieren. Weitere mögliche Ausgestaltungen, Weiterbildungen und Implementierungen der Erfindung umfassen auch nicht explizit genannte Kombinationen von zuvor oder im Folgenden bezüglich der Ausführungsbeispiele beschriebenen Merkmale der Erfindung. Insbesondere wird dabei der Fachmann auch Einzelaspekte als Verbesserungen oder Ergänzungen zu der jeweiligen Grundform der vorliegenden Erfindung hinzufügen.

### INHALTSANGABE DER ZEICHNUNG

Die vorliegende Erfindung wird nachfolgend anhand der in den schematischen Figuren der Zeichnungen angegebenen Ausführungsbeispiele näher erläutert. Es zeigen dabei:
- Fig. 1: eine schematische Draufsicht einer Ausführungsform einer Fluidschlauchvorrichtung;
- Fig. 2: eine schematische Draufsicht einer weiteren Ausführungsform einer Fluidschlauchvorrichtung;
- Fig. 3a, 3b: zwei schematische Schnittansichten einer Ausführungsform einer Fluidschlauchvorrichtung;
- Fig. 4a, 4b: zwei schematische Schnittansichten einer weiteren Ausführungsform einer Fluidschlauchvorrichtung;
- Fig. 5: eine schematische Schnittansicht einer Ausführungsform einer Fluidschlauchvorrichtung;
- Fig. 6: eine schematische Schnittansicht einer Ausführungsform einer Fluidschlauchvorrichtung;
- Fig. 7: eine schematische Schnittansicht einer weiteren Ausführungsform einer Fluidschlauchvorrichtung; und
- Fig. 8: eine schematische Ansicht eines CPAP-Beatmungsgerätes mit einer Ausführungsform einer Fluidschlauchvorrichtung.

Die beiliegenden Zeichnungen sollen ein weiteres Verständnis der Ausführungsformen der Erfindung vermitteln. Sie veranschaulichen Ausführungsformen und dienen im Zusammenhang mit der Beschreibung der Erklärung von Prinzipien und Konzepten der Erfindung. Andere Ausführungsformen und viele der genannten Vorteile ergeben sich im Hinblick auf die Zeichnungen. Die Elemente der Zeichnungen sind nicht notwendigerweise maßstabsgetreu zueinander gezeigt.

In den Figuren der Zeichnung sind gleiche, funktionsgleiche und gleich wirkende Elemente, Merkmale und Komponenten - sofern nichts Anderes ausführt ist -jeweils mit denselben Bezugszeichen versehen.

### BESCHREIBUNG VON AUSFÜHRUNGSBEISPIELEN

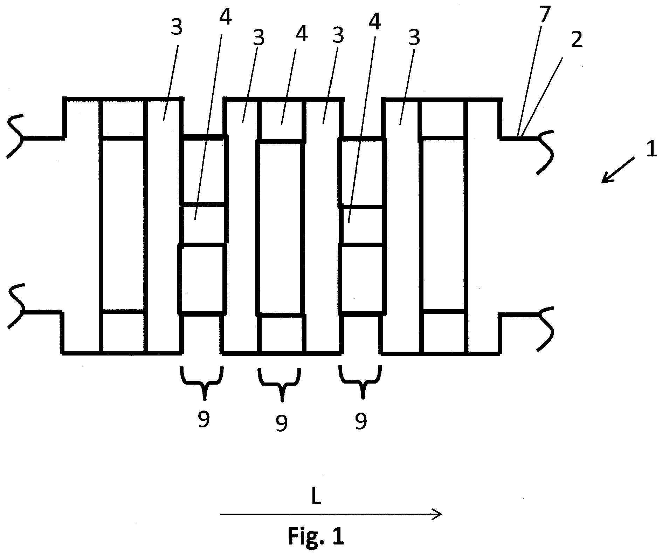

Fig. 1 zeigt eine schematische Draufsicht einer Ausführungsform einer Fluidschlauchvorrichtung 1. Die Fluidschlauchvorrichtung 1 weist einen Fluidschlauch 2 auf, durch welchen ein Fluid transportierbar ist. Das Fluid kann beispielsweise ein Atmengas sein, welches zur Beatmung eines Patienten dient. Der Fluidschlauch kann beispielsweise aus einem thermoplastischen und/oder elastomeren Kunststoff ausgebildet sein.

An dem Fluidschlauch 2 sind eine Vielzahl von Knickschutz-Erhebungen 3, 11 vorgesehen, welche in Längsrichtung L des Fluidschlauches 2 angeordnet sind. Die Knickschutz-Erhebungen sind parallel zueinander und in regelmäßigen Abständen voneinander beabstandet. Die Knickschutz-Erhebungen erstrecken sich kreisförmig um die Manteloberfläche 7 des Fluidschlauches 2 herum. Bei einer Biegung des Fluidschlauches 2 kommen nebeneinander angeordnete miteinander in Anlage, und verhindern dadurch eine weitere Biegung des Fluidschlauches 2.

Zwischen zwei benachbarten Knickschutz-Erhebungen 3, 11 ist eine Gruppe 9 einer Mehrzahl von Verbindungsstegen 4, 10 vorgesehen, die die benachbarten Knickschutz-Erhebungen 3, 11 miteinander verbinden. Erfindungsgemäß sind die Gruppen 9 in einem alternierenden Winkelmuster entlang der Längsrichtung L des Fluidschlauches 1 angeordnet. Auf diese Weise kann die Formstabilität des erhöht und die Knickanfälligkeit vermindert t werden.

Gemäß dieser Ausführungsform umfassen die Gruppen jeweils zwei Verbindungstege 4, 10, welche um 90° oder 180° versetzt zueinander angeordnet sind. Die nebeneinander vorgesehenen Gruppen 9 von Verbindungsstegen können jedoch auch 15°, 30°, 45°, 60°, 105° und/oder 120° zueinander vorgesehen sein.

Allgemein ausgedrückt, können die Gruppen 9 n Verbindungstege 4, 10 umfassen, welche um 360°/n versetzt zueinander angeordnet sind, wobei n eine natürliche Zahl größer oder gleich 3 ist.

Auch können die Gruppen 9 jeweils um 360°/2n winkelversetzt angeordnet sein, wobei n eine natürliche Zahl größer oder gleich 3 ist.

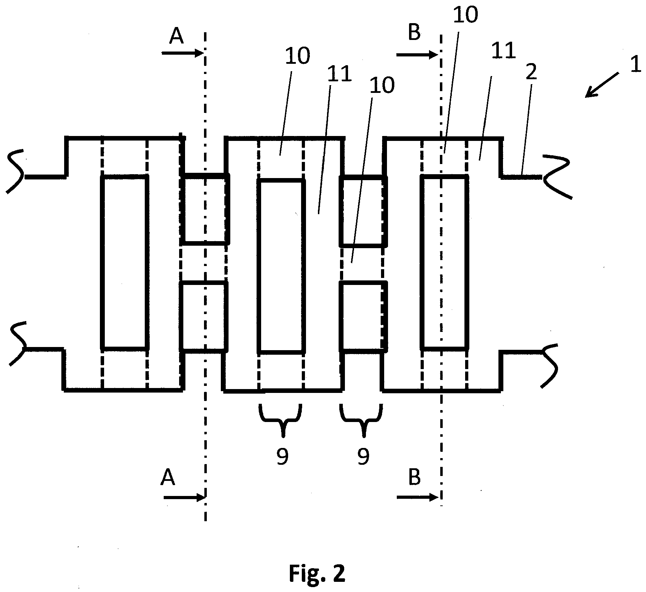

Fig. 2 zeigt eine schematische Draufsicht einer weiteren Ausführungsform einer Fluidschlauchvorrichtung 1. Gemäß dieser Ausführungsform sind die Knickschutz-Erhebungen 3, 11 und/oder die Verbindungsstege 4, 10 einstückig mit dem Fluidschlauch 2 ausgebildet sind. Auf diese Weise kann die Fluidschlauchvorrichtung 1 sehr kostengünstig ausgebildet sein, da die Fluidschlauchvorrichtung in einem Spritzgussverfahren herstellbar ist.

Die einzelnen Knickschutz-Erhebungen 3, 11 sind gemäß diesem Ausführungsbeispiel äquidistant und parallel zueinander angeordnet. Jedoch ist es auch möglich, die Knickschutz-Erhebungen 3 in unregelmäßigen Abständen zueinander anzuordnen, um dadurch die Flexibilität der Fluidschlauchvorrichtung 1 verändern und an bestimmte Randbedingungen anpassen zu können.

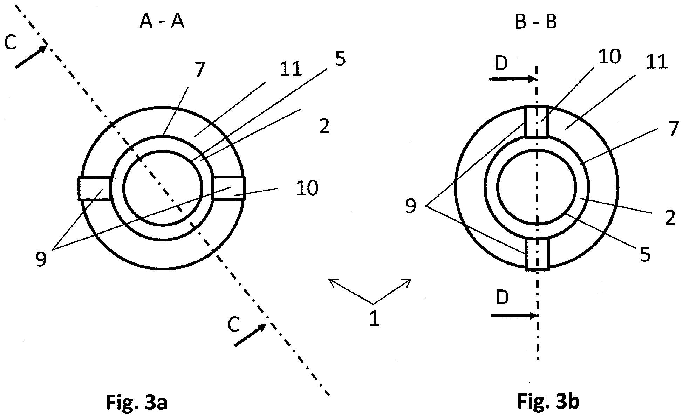

Fig. 3a und 3b zeigen zwei schematische Schnittansichten einer Ausführungsform einer Fluidschlauchvorrichtung 1. Der Verlauf der Schnitte A - A und B - B ist in der Fig. 2 dargestellt. Wie aus Fig. 3a und 3b ersichtlich, sind die benachbarten Gruppen 9 von Verbindungsstegen 10 winkelmässig zueinander versetzt angeordnet. In dem gezeigten Ausführungsbeispiel sind es 90°. Das gezeigte Ausführungsbeispiel weist pro Gruppe 9 zwei Verbindungsstege 4 auf, welche die nebeneinander angeordneten Knickschutz-Erhebungen 3 miteinander verbinden. Die Verbindungsstege sind gegenüberliegend am Fluidschlauch 2 vorgesehen.

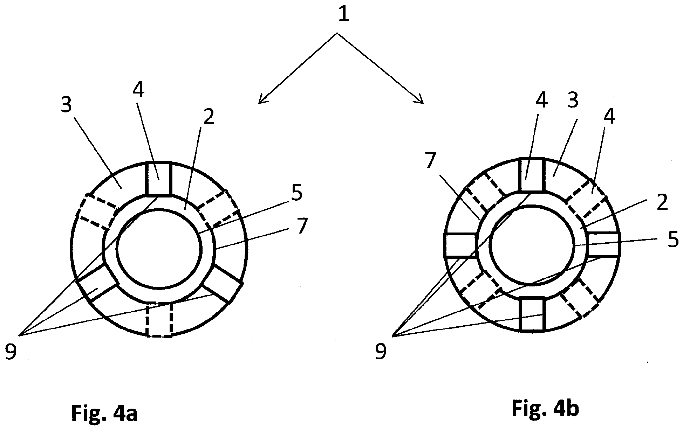

Fig. 4a und 4b zeigen zwei schematische Schnittansichten jeweils einer Ausführungsform einer Fluidschlauchvorrichtung 1. Die Fig. 4a zeigt eine Fluidschlauchvorrichtung 1, bei der eine Gruppe 9 jeweils drei Verbindungsstege umfasst, welche jeweils zueinander um 120° versetzt angeordnet sind. Zwei in Längsrichtung des Fluidschlauches 2 nebeneinander vorgesehene Gruppen sind dann um beispielsweise 60° zueinander versetzt angeordnet. In der Fig. 4a ist die nachfolgende Gruppe an Verbindungsstegen durch gestrichelte Linien dargestellt. Die Fig. 4b zeigt eine Fluidschlauchvorrichtung 1, bei der eine Gruppe 9 jeweils vier Verbindungsstege umfasst, welche jeweils zueinander um 90° versetzt angeordnet sind. Zwei in Längsrichtung des Fluidschlauches 2 nebeneinander vorgesehene Gruppen sind dann um beispielsweise 45° zueinander versetzt angeordnet. In der Fig. 4b ist die nachfolgende Gruppe an Verbindungsstegen 4 durch gestrichelte Linien dargestellt.

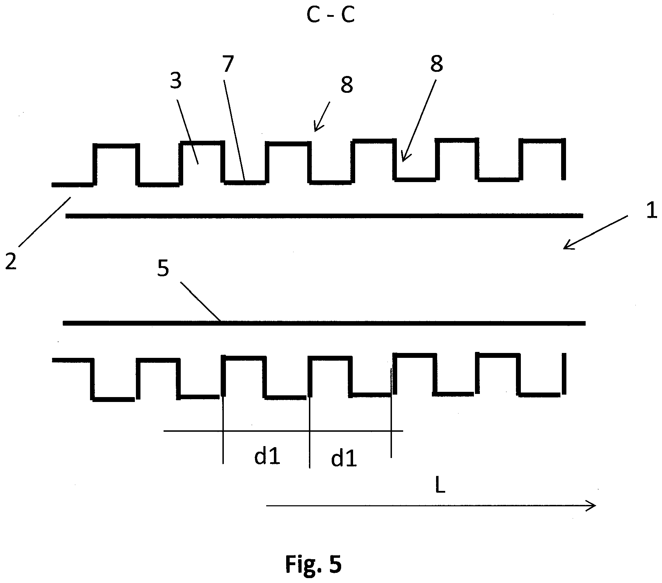

Fig. 5 zeigt eine schematische Schnittansicht einer Ausführungsform einer Fluidschlauchvorrichtung 1. Der Verlauf des Schnitts C -C ist in Fig. 3a dargestellt. Die Knickschutz-Erhebungen 3 bilden ein kammförmiges Muster entlang der Längsrichtung L des Fluidschlauches. Ferner erkennt man in dieser Fig., dass die einzelnen Kickschutzerhebungen 3 denselben Abstand d1 jeweils zueinander aufweisen.

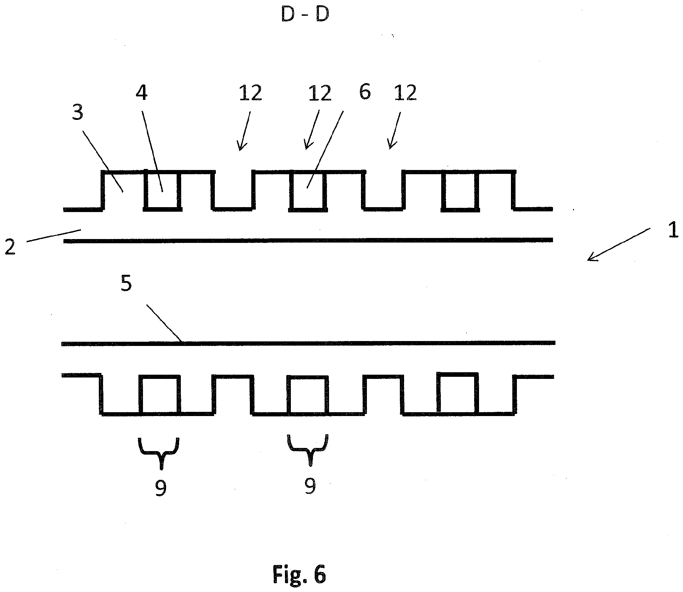

Fig. 6 zeigt eine schematische Schnittansicht einer Ausführungsform einer Fluidschlauchvorrichtung 1. Der Verlauf des Schnitts D - D ist in Fig. 3b dargestellt, jedoch kann sich diese Ausführungsform von der in Fig. 3b dargestellten Ausführungsform unterscheiden. In Fig. 3b weisen die Verbindungsstege zugfeste Fasern 6 auf, welche die Zugfestigkeit der Verbindungsstege 4 erhöhen. Die zugfesten Fasern 6 können beispielsweise aus einem Kunststoff ausgebildet sein. Jedoch können auch Glasfasern, Aramidfasern, Keramikfasern oder Kohlenstofffasern verwendet werden.

Auch ist es, wie in diesem Ausführungsbeispiel ersichtlich, dass die Gruppen 9 von Verbindungsstegen in jedem zweiten Spalt zwischen zwei Knickschutz-Erhebungen 3 vorgesehen sind. Auch ist es möglich, dass die Verbindungsstege 4 nur in jeder dritten Spalte oder vierten Spalte angeordnet sind.

Die Fluidschlauchvorrichtung 1 kann auch derart ausgebildet sein, dass der Fluidschlauch 2 aus einem ersten Kunststoff ausgebildet ist und die Knickschutz-Erhebungen 3 und die Verbindungsstege 4 aus einem zweiten, vom ersten Kunststoff verschiedenen Kunststoff ausgebildet sind. Beispielsweise können die Knickschutz-Erhebungen und die Verbindungsstege aus einem im Vergleich zum ersten Kunststoff zugfesteren Kunststoff ausgebildet sein.

Des Weiteren, wie beispielsweise aus Fig. 6 ersichtlich, ist die Innenseite 5 des Fluidschlauchs 2 im Wesentlichen eben ausgebildet. Jedoch kann die Innenseite 5 des Fluidschlauchs 2 auch mit einem Muster versehen werden

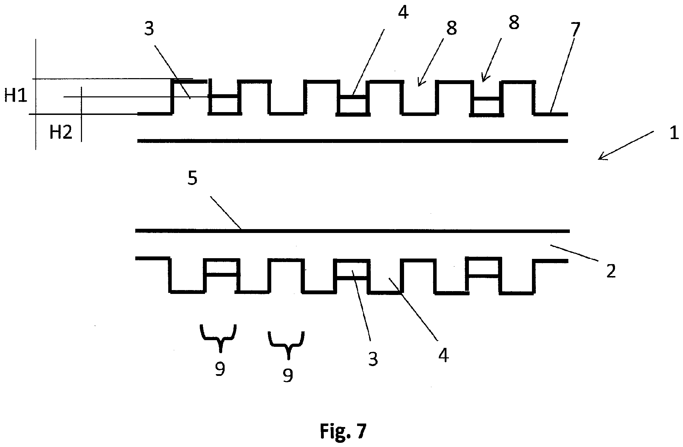

Fig. 7 zeigt eine schematische Schnittansicht einer weiteren Ausführungsform einer Fluidschlauchvorrichtung 1. Im Gegensatz zu den in den Fig. 1 - 6 gezeigten Ausführungsbeispielen, bei denen sich die Knickschutz-Erhebungen 3, 11 und die Verbindungstege 4, 10 auf eine gleiche Höhe ausgehend von der Manteloberfläche 7 des Fluidschlauches 2 erstrecken, erstrecken sich die die Knickschutz-Erhebungen 3, 11 und die Verbindungstege 4, 10 nicht auf eine gleiche Höhe ausgehend von der Manteloberfläche 7 des Fluidschlauches 2. Auf diese Weise ist es möglich, die Flexibilität und die Formstabilität an die jeweiligen Randbedingungen anzupassen. Je höher die Verbindungsstege sich erstrecken, desto höher ist die Formstabilität der Fluidschlauchvorrichtung 1.

Im dargestellten Ausführungsbeispiel erstrecken sich die Verbindungsstege auf eine Höhe H2, welche geringer ist als die Höhe H1 der Knickschutz-Erhebungen.

Ferner weisen in diesem Ausführungsbeispiel die Knickschutz-Erhebungen 3 und/oder die Verbindungsstege 4 abgerundete Außenseiten auf. Auf diese Weise kann die Lebensdauer der Fluidschlauchvorrichtung verlängert werden, da abgerundete Außenseiten weniger anfällig für Rissbildung sind. Die abgerundeten Außenseiten können beispielsweise einen Radius von 0,1 mm bis 2 mm, vorzugsweise 0,3 mm aufweisen.

Die Knickschutz-Erhebungen 3 und die Verbindungsstege 4 sind derart ausgebildet, dass die Fluidschlauchvorrichtung 1 ein winkelunabhängiges Biegeverhalten aufweist. Dies wird dadurch erreicht, dass die Verbindungsstege in einem gleichmäßigen Muster entlang der Längsrichtung L der Fluidschlauchvorrichtung 1 vorgesehen sind.

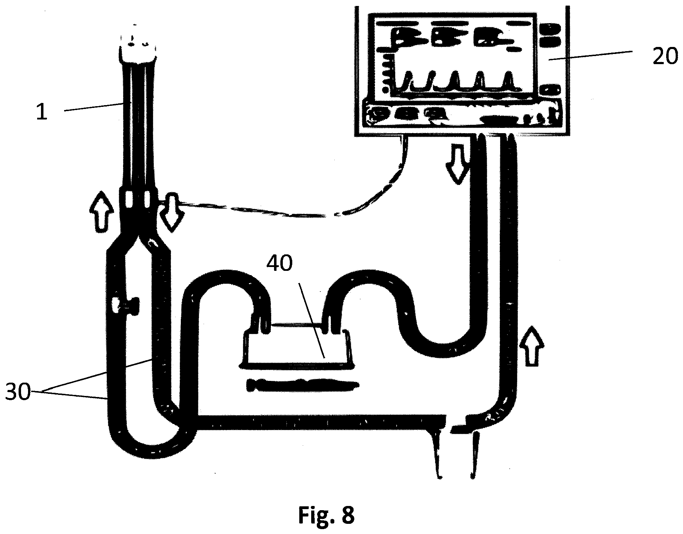

Fig. 8 zeigt eine schematische Ansicht eines CPAP-Beatmungsgerätes 20. Das CPAP-(Continuous Positive Airway Pressure)-Beatmungsgerät 20 ist eine Beatmungsform, weist verschiedene Fluidleitungen 30 auf, welche ähnlich der Fluidschlauchvorrichtung 1 gemäß der Erfindung ausgebildet sein können. Ferner weist das CPAP-Gerät 20 einen Befeuchter 40 auf, welcher das Atemgas befeuchtet. Des Weiteren weist das CPAP-Beatmungsgerät 20 eine Fluidschlauchvorrichtung 1 gemäß einem Ausführungsbeispiel der vorliegenden Erfindung auf. Die Fluidschlauchvorrichtung kann beispielsweise an eine Atemmaske angekoppelt sein, und über die Atemmaske den Patienten Atemgas zuführen oder Atemgas zurückleiten.

Obwohl die vorliegende Erfindung anhand bevorzugter Ausführungsbeispiele vorstehend vollständig beschrieben wurde, ist sie darauf nicht beschränkt, sondern auf vielfältige Art und Weise modifizierbar.

### BEZUGSZEICHENLISTE

- 1: Fluidschlauchvorrichtung
- 2: Fluidschlauch
- 3: Knickschutz-Erhebungen
- 4: Verbindungsstege
- 5: Innenseite Fluidschlauch
- 6: Zugfeste Fasern
- 7: Manteloberfläche des Fluidschlauchs
- 8: abgerundete Ecken
- 9: Gruppe
- 10: Verbindungsstege
- 11: Knickschutz-Erhebungen
- 12: Spalten

- 20: Beatmungsvorrichtung
- 30: Fluidschläuche
- 40: Befeuchter

## Patentansprüche

1. Fluidschlauchvorrichtung (1),
mit einem Fluidschlauch (2), durch welchen ein Fluid transportierbar ist,
mit einer Vielzahl von in Längsrichtung (L) des Fluidschlauches (1) vorgesehenen, voneinander beabstandeten Knickschutz-Erhebungen (3, 11), welche sich kreisförmig um die Manteloberfläche (7) des Fluidschlauches (2) herum erstrecken,
**dadurch gekennzeichnet, dass** jeweils zwischen zwei benachbarten Knickschutz-Erhebungen (3, 11) eine Gruppe (9) einer Mehrzahl von Verbindungsstegen (4, 10) vorgesehen ist, die die benachbarten Knickschutz-Erhebungen (3, 11) miteinander verbinden, und
die Gruppen (9) in einem alternierenden Winkelmuster entlang der Längsrichtung (L) des Fluidschlauches (1) angeordnet sind.

2. Fluidschlauchvorrichtung (1) nach Anspruch 1,
**dadurch gekennzeichnet,**
**dass** die Gruppen (9) zwei Verbindungstege (4, 10) umfassen, welche um 180° versetzt zueinander angeordnet sind.

3. Fluidschlauchvorrichtung (1) nach Anspruch 2,
**dadurch gekennzeichnet,**
**dass** die Gruppen (9) jeweils um 90° Grad winkelversetzt angeordnet sind.

4. Fluidschlauchvorrichtung (1) nach Anspruch 1,
**dadurch gekennzeichnet,**
**dass** die Gruppen (9) n Verbindungstege (4, 10) umfassen, welche um 360°/n versetzt zueinander angeordnet sind, wobei n eine natürliche Zahl größer oder gleich 3 ist.

5. Fluidschlauchvorrichtung (1) nach Anspruch 2,
**dadurch gekennzeichnet,**
**dass** die Gruppen (9) jeweils um 360°/2n winkelversetzt angeordnet sind, wobei n eine natürliche Zahl größer oder gleich 3 ist.

6. Fluidschlauchvorrichtung (1) nach einem der vorhergehenden Ansprüche,
**dadurch gekennzeichnet,**
**dass** die Knickschutz-Erhebungen (3, 11) und/oder die Verbindungsstege (4, 10) einstückig mit dem Fluidschlauch (2) ausgebildet sind.

7. Fluidschlauchvorrichtung (1) nach einem der vorhergehenden Ansprüche,
**dadurch gekennzeichnet,**
**dass** die Knickschutz-Erhebungen (3, 11) äquidistant und parallel angeordnet sind.

8. Fluidschlauchvorrichtung (1) nach einem der vorhergehenden Ansprüche,
**dadurch gekennzeichnet,**
**dass** sich die Knickschutz-Erhebungen (3, 11) und die Verbindungstege (4, 10) auf eine gleiche Höhe ausgehend von der Manteloberfläche (7) des Fluidschlauches (2) erstrecken.

9. Fluidschlauchvorrichtung (1) nach einem der vorhergehenden Ansprüche,
**dadurch gekennzeichnet,**
**dass** der Fluidschlauch (2) ein Kunststoffspritzgussteil aus einem thermoplastischen Kunststoff, z. B. TPE-S, ist.

10. Fluidschlauchvorrichtung (1) nach einem der vorhergehenden Ansprüche,
**dadurch gekennzeichnet,**
**dass** die Innenseite (5) des Fluidschlauchs (2) im Wesentlichen eben ausgebildet ist.

11. Fluidschlauchvorrichtung (1) nach einem der vorhergehenden Ansprüche,
**dadurch gekennzeichnet,**
**dass** die Verbindungsstege (4, 10) zugfeste Fasern (6) aufweisen.

12. Fluidschlauchvorrichtung (1) nach einem der vorhergehenden Ansprüche,
**dadurch gekennzeichnet,**
**dass** der Fluidschlauch (2) aus einem ersten Kunststoff ausgebildet ist und die Knickschutz-Erhebungen (3, 11) und die Verbindungsstege (4, 10) aus einem zweiten, vom ersten Kunststoff verschiedenen Kunststoff ausgebildet sind.

13. Fluidschlauchvorrichtung (1) nach einem der vorhergehenden Ansprüche,
**dadurch gekennzeichnet,**
**dass** die Knickschutz-Erhebungen (3, 11) und/oder die Verbindungsstege (4, 10) abgerundete Außenseiten aufweisen.

14. Fluidschlauchvorrichtung (1) nach einem der vorhergehenden Ansprüche,
**dadurch gekennzeichnet**,
die Knickschutz-Erhebungen (3, 11) und die Verbindungsstege (4, 10) derart ausgebildet sind, dass die Fluidschlauchvorrichtung (1) ein winkelunabhängiges Biegeverhalten aufweist.

## Claims

1. Fluid hose device (1),
comprising a fluid hose (2) through which a fluid is transportable,
comprising a plurality of kink-protection elevations (3, 11), which are provided in the longitudinal direction (L) of the fluid hose (1), are spaced apart, and extend in a circle shape around the outer surface (7) of the fluid hose (2),
**characterised in that**, between every two adjacent kink-protection elevations (3, 11), a group (9) of a plurality of connecting webs (4, 10), which interconnect the adjacent kink-protection elevations (3, 11), is provided, and
the groups (9) are arranged in an alternating angle pattern along the longitudinal direction (L) of the fluid hose (1).

2. Fluid hose device (1) according to claim 1,
**characterised in that**
the groups (9) comprise two connecting webs (4, 10) which are arranged offset through 180° with respect to one another.

3. Fluid hose device (1) according to claim 2,
**characterised in that**
the groups (9) are arranged angularly offset through 90° in each case.

4. Fluid hose device (1) according to claim 1,
**characterised in that**
the groups (9) comprise n connecting webs (4, 10) which are arranged offset through 360°/n with respect to one another, n being a natural number greater than or equal to 3.

5. Fluid hose device (1) according to claim 2,
**characterised in that**
the groups (9) are arranged angularly offset through 360°/2n in each case, n being a natural number greater than or equal to 3.

6. Fluid hose device (1) according to any of the preceding claims,
**characterised in that**
the kink-protection elevations (3, 11) and/or the connecting webs (4, 10) are formed integrally with the fluid hose (2).

7. Fluid hose device (1) according to any of the preceding claims,
**characterised in that**
the kink-protection elevations (3, 11) are arranged equidistant and parallel.

8. Fluid hose device (1) according to any of the preceding claims,
**characterised in that**
the kink-protection elevations (3, 11) and the connecting webs (4, 10) extend to the same height from the outer surface (7) of the fluid hose (2).

9. Fluid hose device (1) according to any of the preceding claims,
**characterised in that**
the fluid hose (2) is a plastics material injection-moulded part made of a thermoplastic plastics material, for example TPE-S.

10. Fluid hose device (1) according to any of the preceding claims,
**characterised in that**
the inner face (5) of the fluid hose (2) is formed substantially smooth.

11. Fluid hose device (1) according to any of the preceding claims,
**characterised in that**
the connecting webs (4, 10) comprise high-tensile-strength fibres (6).

12. Fluid hose device (1) according to any of the preceding claims,
**characterised in that**
the fluid hose (2) is formed from a first plastics material, and the kink-protection elevations (3, 11) and the connecting webs (4, 10) are formed from a second plastics material different from the first plastics material.

13. Fluid hose device (1) according to any of the preceding claims,
**characterised in that**
the kink-protection elevations (3, 11) and/or the connecting webs (4, 10) comprise rounded outer faces.

14. Fluid hose device (1) according to any of the preceding claims,
**characterised in that**
the kink-protection elevations (3, 11) and the connecting webs (4, 10) are formed in such a way that the fluid hose device (1) has angle-independent bending properties.

## Revendications

1. Dispositif de tuyau à fluide (1),
comprenant un tuyau à fluide (2) à travers lequel un fluide est transportable,
comportant une pluralité d'élévations (3 ,11) de protection anti-pli distancées les unes des autres, ménagées dans le sens longitudinal (L) du tuyau à fluide (1), lesquelles s'étendent de manière circulaire autour de la surface d'enveloppe (7) du tuyau à fluide (2), **caractérisé en ce qu'**entre deux élévations (4, 10) adjacentes de protection anti-pli, un groupe (9) d'une pluralité de nervures de liaison (4, 10) est respectivement ménagé, lesquelles relient entre elles les élévations (3, 11) de protection anti-pli, et
**en ce que** les groupes (9) sont disposés dans un modèle angulaire alternant le long du sens longitudinal (L) du tuyau à fluide (1).

2. Dispositif de tuyau à fluide (1) selon la revendication 1, **caractérisé en ce,**
**que** les groupes (9) comprennent deux nervures de liaison (4, 10) qui sont disposées de manière décalée de 180° l'une par rapport à l'autre.

3. Dispositif de tuyau à fluide (1) selon la revendication 2,
**caractérisé en ce**
**que** les groupes (9) sont disposés en angle décalé de respectivement 90° degrés.

4. Dispositif de tuyau à fluide (1) selon la revendication 1, **caractérisé en ce**
**que** les groupes (9) comprennent n nervures de liaison (4, 10) lesquelles sont disposées de manière décalée de 360°/n les unes par rapport aux autres, n étant un nombre naturel supérieur ou égal à 3.

5. Dispositif de tuyau à fluide (1) selon la revendication 2, **caractérisé en ce**
**que** les groupes (9) sont disposés de manière décalée de respectivement 360°/2n les uns par rapport aux autres, n étant un nombre naturel supérieur ou égal à 3.

6. Dispositif de tuyau à fluide (1) selon l'une quelconque des revendications précédentes,
**caractérisé en ce**
**que** les élévations (3, 11) de protection anti-pli et/ou les nervures de liaison (4, 10) sont réalisées d'une seule pièce avec le tuyau à fluide (2).

7. Dispositif de tuyau à fluide (1) selon l'une quelconque des revendications précédentes,
**caractérisé en ce**
**que** les élévations (3, 11) de protection anti-pli sont disposées de manière équidistante et parallèle.

8. Dispositif de tuyau à fluide (1) selon l'une quelconque des revendications précédentes,
**caractérisé en ce**
**que** les élévations (3, 11) de protection anti-pli et les nervures de liaison (4, 10) s'étendent sur une même hauteur à partir de la surface d'enveloppe (7) du tuyau à fluide (2).

9. Dispositif de tuyau à fluide (1) selon l'une quelconque des revendications précédentes,
**caractérisé en ce**
**que** le tuyau à fluide (2) est une pièce en matière plastique moulée par injection constituée d'une matière thermoplastique, par ex. TPE-S.

10. Dispositif de tuyau à fluide (1) selon l'une quelconque des revendications précédentes,
**caractérisé en ce**
**que** le côté intérieur (5) du tuyau à fluide (2) est essentiellement réalisé de manière plane.

11. Dispositif de tuyau à fluide (1) selon l'une quelconque des revendications précédentes,
**caractérisé en ce**
**que** les nervures de liaison (4, 10) présentent des fibres (6) résistantes à la traction.

12. Dispositif de tuyau à fluide (1) selon l'une quelconque des revendications précédentes,
**caractérisé en ce**
**que** le tuyau à fluide (2) est réalisé dans une première matière plastique et en ce que les élévations (3, 11) de protection anti-pli et les nervures de liaison (4, 10) sont réalisées dans une deuxième matière plastique différente de la première matière plastique.

13. Dispositif de tuyau à fluide (1) selon l'une quelconque des revendications précédentes,
**caractérisé en ce**
**que** les élévations (3, 11) de protection anti-pli et/ou les nervures de liaison (4, 10) présentent des côtés extérieurs arrondis.

14. Dispositif de tuyau à fluide (1) selon l'une quelconque des revendications précédentes,
**caractérisé en ce**
**que** les élévations (3, 11) de protection anti-pli et les nervures de liaison (4, 10) sont réalisées de manière à ce que le dispositif de tuyau à fluide (1) présente un comportement en flexion indépendant de l'angle.
